# EUROPEAN PATENT APPLICATION

(11) **EP 1 695 695 A1**
(43) Date of publication of application: **30.08.2006**
(21) Application number: 06000838.0
(22) Date of filing: 16.01.2006
(51) Int. Cl.: A61K 9/107

(54) **Water-in-oil type micro-emulsion**

(30) Priority: 25.02.2005 JP 2005051220
(71) Applicant: DAI-ICHI KOGYO SEIYAKU CO., LTD., Shimogyo-ku Kyoto (JP)
(72) Inventor: Mori, Toshiki, Higashiomi-shi Shiga 529-1551 (JP)
(74) Representative: Strehl Schübel-Hopf & Partner

(57) **Abstract**

A water-oil type micro-emulsion having high safety to a living body by decreasing the amount of a surfactant to be used or not using the surfactant, and at a high ratio of aqueous phase capable of dissolving effective ingredients, the water-in-oil type micro-emulsion comprising polyvinyl pyrrolidone and one or more of members selected from the group consisting of fatty acids which are liquid at a normal temperature and higher alcohols which are liquid at a normal temperature.

## Description

### BACKGROUND OF THE INVENTION

### Field of the Invention

The present invention concerns a water-in-oil micro-emulsion.

### Description of the Related Art

A micro-emulsion is a system in which extremely fine liquid droplets with a grain size of about from 10 to 100 nm are dispersed and can be formed by properly selecting the ratio of water: oil: surfactant to be used, a combination of characteristics of the surfactant and the oil to be used, or by using a medium-chained alcohol, for example, butanol, heptanol, octanol, etc. while this is different depending on the concentration of salt and pH of water to be used, the extent of polarity or the chemical structure of an oil, HLB or the chemical structure of a surfactant.

Since the liquid droplets of the microemulsion are extremely fine particles as described above, an attempt of utilizing them for drug delivery systems (DDS) has been found occasionally in recent years. Specifically, it has been attempted to utilize the micro-emulsion for, the drug delivery system of physiologically active substances that are less absorbed percutaneously/permucosally, for example, physiologically active peptides of high molecular weight and less absorbing low molecular compounds. That is, since most of such high molecular weight peptides are water soluble and most of the less absorbing low molecular weight compounds are also water soluble, it has been attempted to improve the absorptivity by formulating as water-in-oil type (hereinafter also referred to as "W/O" type) micro-emulsion (JP-A No. 7-2689, "Oleoscience" Vol. 1, No. 7, 743 (2001)).

However, compared with the oil-in-water type (O/W type) micro-emulsions there are few cases of studies for W/O type micro-emulsions and, in most of them, aerosol OT (AOT: sodium di-2-ethylhexyl sulfo succinate) was used.

### SUMMARY OF THE INVENTION

Since the use of a great amount of surfactant has been required in the existent W/O micro-emulsions described above, it gives rise to a problem in view of the safety. For example, in Example 1 shown in JP-A-7-2689, 12 g of a surfactant is used for solubilizing 2 g of aqueous phase.

Further, AOT mainly used so far involves a problem of low safety and biocompatibility when applied as medicines and cosmetics.

The invention has been achieved in view of the foregoings and it intends to provide a water-in-oil type micro-emulsion having high safety to a living body since the amount of the surfactant to be used is decreased or the surfactant is not used.

For solving the foregoing problem, the micro-emulsion in accordance with the invention is a water-in-oil type micro-emulsion containing polyvinyl pyrrolidone and one or more of members selected from the group consisting of a fatty acids which are liquid at a normal temperature and higher alcohols which are liquid at a normal temperature.

The micro-emulsion according to the invention described above may contain one or more of members selected from the group consisting of polyhydric alcohols, inorganic salts, organic acid salts, amino acid, amino acid salts in an aqueous phase as a second feature.

The micro-emulsion according to the invention may further contain a surfactant as a third feature.

According to the invention, since the amount of the surfactant to be used is decreased or the surfactant is not used, it can provide a water-in-oil type micro-emulsion of high safety to a living body.

According to the second feature of the invention, it is also possible to provide a water-in-oil type micro-emulsion with a high ratio of the aqueous phase capable of dissolving chemicals.

### DESCRIPTION OF PREFERRED EMBODIMENTS

Each of the ingredients constituting the micro-emulsion according to the invention is to be described below.

### < Polyvinyl pyrrolidone >

Polyvinyl pyrrolidone is a polymer of N-vinyl-2-vinylpyrrolidone which can be produced by known methods and commercially available products can also be used. For the molecular weight, polymers with Mw of 500,000 or less are preferably used with a view point of giving less effect on the viscosity of systems.

The blending amount of polyvinyl pyrrolidone is preferably from 0.1% by weight to 40% by weight and, more preferably, from 5% by weight to 15% by weight based on the entire system. In a case where it is less than 0. 1% by weight, the effect is insufficient. In a case where it exceeds 40% by weight, the viscosity of the system increases excessively to worsen the property in use.

While polyvinyl pyrrolidone may be incorporated only in one of the oil phase and the aqueous phase upon preparation of the emulsion, formation of the emulsion is more facilitated by incorporating the same into both of the oil phase and the aqueous phase.

### < Fatty acid which is liquid at normal temperature and/or higher alcohol which is liquid at normal temperature >

The fatty acid and the higher alcohol are not particularly limited so long as they are liquid at a normal temperature (25°C), and those of 6 or more carbon atoms are preferred. They include, for example, linear fatty acids such as capronic acid, and caprilic acids, branched fatty acids such as isopalmitic acid, and isostearic acid, unsaturated fatty acids such as oleic acid, myristoleic acid, and palmitoleic acid, linear higher alcohols such as octanol and decanol, branched higher alcohols such as isostearyl alcohol and octyl dodecanol, and unsaturated higher alcohols such as oleyl alcohol. In view of the safety, oleic acid is particularly preferred.

For the fatty acid and the higher alcohol, one or more types of either fatty acids or higher alcohols may be used, or one or more of fatty acids and one or more of higher alcohols may be used together.

The fatty acid and/or higher alcohol is used, preferably, by 20% by weight or more and, more preferably, by 50% by weight or more in the oil phase ingredient. The oil phase may also be formed only with fatty acid and/or higher alcohol without adding other oil phase ingredient. In a case of using other oil phase ingredient, liquid paraffin, fatty acid triglyceride such as triglyceride ethyl hexylate, esters of lower alcohols and higher fatty acids such as isopropyl palmitate can be used.

### < Polyhydric alcohol >

Examples of the polyhydric alcohol used in the invention include propylene glycol, glycerine, sorbitol, etc., and tri-or higher polyhydric alcohols such as glycerine and sorbitol are preferred.

By the use of such polyhydric alcohols or salts, amino acids to be describe later, solubility of polyvinyl pyrrolidone to the aqueous phase can be controlled and the ratio of the aqueous phase can be increased.

The blending amount of the polyhydric alcohol is preferably 90% by weight or less at a ratio in the aqueous phase ingredient. In a case where it exceeds 90% by weight, the blending amount of water is decreased relatively and it may happens that the water soluble active substance can not be dissolved sufficiently.

### < Inorganic salt, organic acid salt, amino acid, amino acid salt >

The inorganic salt, organic acid salt, amino acid, and amino acid salt used in the invention are not particularly limited so long as they are water soluble and show salting out effect due to dehydration effect and they include, for example, chlorides such as sodium chloride, sulfates such as sodium sulfate, citrates such as monosodium citrate, disodium citrate, trisodium citrate, and monopotassium citrate, succinates such as monosodium succinate and disodium succininate, carbonates such as sodium carbonate and sodium hydrogen carbonate, phosphates such as sodium phosphate and sodium hydrogen phosphate, and glycine, proline, glutamic acid and sodium glutamic acid.

The blending amount of them is preferably 50% by weight or less, more preferably, from 10% by weight to 40% by weight as a ratio in the aqueous phase ingredient while depending on the solubility thereof. In a case where it exceeds 50% by weight, salting out, etc. occurs tending to render the system instable.

### < Surfactant >

In the emulsion according to the invention, a surfactant can be added within a range not deteriorating the function such as safety. Preferred surfactants are nonionic surfactants such as sucrose fatty acid ester, glycerine fatty acid ester, polyglicerine fatty acid ester, sorbitan fatty acid ester, polyoxyethylene fatty acid ester, polyoxyethylene hardened castor oil, polyoxyethylene alkyl ether, polyoxyethylene fatty acid ester, etc. are preferred.

The blending amount of the surfactant is 3:1 or less, preferably, 1:1 or less based on the aqueous phase expressed as the weight ratio.

The surfactant may be incorporated in one of the oil phase and the aqueous phase or may be incorporated in both of them upon preparation of the emulsion.

### < Physiologically active substance >

Physiological active substances with low percutaneous/permucosa absorptivity applicable to the invention are not particularly limited and include for example, peptide chemicals such as vasopressin, calcitonin, erythropoietin, collony stimulative factors, interleukins, interferons, insulin, and parathyroid hormone; and less absorbing low molecular chemicals with a molecular weight of 1000 or less. Examples of the low molecular weight chemicals include, for example, antibiotics (aclarubicin hydrochloride, oxytetracycline hydrochloride, cefotiam hydrochloride, calbenicillin sodium, cefmetazole sodium, etc.), antiarrhythmics (procain amide hydrochloride, disopyramide phosphate, lidocaine hydrochloride, etc.), cardiotonics (etirefline hydrochloride, dopamine hydrochloride, etc.), vasodilators (trapidil, etc.), topical anesthetics (oxybuprocaine hydrochloride, dibucaine hydrochloride, procaine hydrochloride, etc.), antineoplastic agents (bleomycin hydrochloride, cytarabine, procarbazine hydrochloride, cisplatin, vinblastine hydrochloride, neocalzinostatin, doxorubicin hydrochloride, etc.), automatic nerves (distigmine bromide, bethanechol chloride, propantheline bromide, etc.), antipyretic analgesics antiphlogistics (antipyrine, tiaramide hydrochloride, diclofenac sodium, etc.), psychoneurosis drugs (imipramine hydrochloride, clomiplamine hydrochloride, thioridazine hydrochloride, fulurazepam hydrochloride, chloropromazine hydrochloride, levomapromazine hydrochloride), narcotic analgesic-cough suppressants (oxycodon hydrochloride, etc.), anticonvulsants (cyclopentorate hydrochloride, etc.), antiparkinson drugs (amantadine hydrochloride, promethadine hydrochloride, metixene hydrochloride, etc.), circulatory drugs (diltiazem hydrochloride, trimetadicine hydrochloride, etc.), antihypertensives (dihydroergotoxin mesylae, clonidine hydrochloride, etc.), enzymatic preparatiaons (urokinase, hyaluronidase, etc.), as well as naphazoline hydrochloride, meclofenaxate hydrochloride, methylephedrin hydrochloride, homatropine hydrobromide etc.

The micro-emulsion of the invention can be produced by the methods known so far. That is, it may be produced by preparing an oil phase and an aqueous phase respectively and adding the aqueous phase while stirring the oil phase. The oil phase may be added optionally to control the content of the effective ingredient. Thus, a micro-emulsion with a grain size of the liquid dispersion droplets of about 10 to 100 nm can be obtained easily.

The micro-emulsion of the invention can be in a gelled form by a water soluble gelling agent. The water soluble gelling agent is, for example, gelatin. The gelled micro-emulsion can be prepared by a known method, for example, as described in "Oleoscience" Vol. 1, No. 7, 743 (2001).

### [Example]

Examples of the invention are to be shown below but the invention is not restricted to the following examples.

Each of the ingredients constituting the oil phase and the aqueous phase were blended respectively at the blending ratio (parts by weight) shown in Table 1 and they were homogenized.

**[Table 1]**

| | | Example 1 | Example 2 | Example 3 | Example 4 |
|---|---|---|---|---|---|
| Oil phase | Polyvinyl pyrrolidone (K-30) | 10 | 10 | 10 | 6 |
| | Oleic acid | 70 | - | 35 | 54 |
| | Oleyl alcohol | - | 70 | - | - |
| | Liquid paraffin | - | - | 35 | - |
| | Sorbitan monooleate | 5 | 5 | 5 | - |
| Aqueous phase | Purified water | 6 | 6 | 6 | 4.2 |
| | Glycerine | 9 | 9 | 9 | 29 |
| | Sodium glutamate | - | - | - | 2.8 |
| | Polyvinyl pyrrolidone (K-30) | - | - | - | 4 |

A micro-emulsion having uniform and transparent appearance was obtained by adding the aqueous phase while stirring the oil phase.

The micro-emulsion according to the invention is utilizable suitably to drugs and the cosmetics as percutaneous, peroral or permucosa administrating agents.

## Claims

1. A water-in-oil type micro-emulsion containing polyvinyl pyrrolidone and one or more of members selected from the group consisting of fatty acids which are liquid at a normal temperature and higher alcohols which are liquid at normal temperature.

2. A water-in-oil type micro-emulsion according to claim 1, wherein one or more of members selected from the group consisting of polyhydric alcohols, inorganic salts, organic acid salts, amino acids, and amino acid salts are contained in an aqueous phase.

3. A water-in-oil type micro-emulsion according to claim 1 or 2, wherein a surfactant is further contained.
